# EUROPÄISCHE PATENTSCHRIFT

(11) **EP 2 796 164 B1**
(45) Veröffentlichungstag und Bekanntmachung des Hinweises auf die Patenterteilung: **10.06.2015**
(21) Anmeldenummer: 13165529.2
(22) Anmeldetag: 26.04.2013
(51) Int. Cl.: A61M 37/00

(54) **Tätowiermaschine zum Einbringen von Tätowierfarbe in menschliche Haut**
Tattoo gun for introducing tattooing ink into human skin
Machine à tatouer pour l'application d'une couleur de tatouage dans la peau humaine

(43) Veröffentlichungstag der Anmeldung: 29.10.2014
(73) Patentinhaber: DC-TP Europe GmbH, 76776 Neuburg am Rhein (DE)
(72) Erfinder: Altenhövel, Florian, 38678 Clausthal-Zellerfeld (DE)
(74) Vertreter: Durm & Partner

(56) Entgegenhaltungen:
- EP-A1- 1 958 659
- FR-A1- 2 972 933

## Beschreibung

Die vorliegende Erfindung betrifft eine Tätowiermaschine zum Einbringen von Tätowierfarbe in menschliche Haut mithilfe einer Tätowiernadel, umfassend, einen Antriebsmotor zum Antreiben der Tätowiernadel, eine Getriebeeinheit und eine Nadelaufnahme zur Aufnahme der Tätowiernadel. Die Getriebeeinheit wandelt eine Antriebsbewegung des Antriebsmotors in eine Stichbewegung der Tätowiernadel, mit der diese in die menschliche Haut eindringt. Die Getriebeheinheit weist einen Kipphebel auf, der durch die Antriebsbewegung des Antriebsmotors eine Kippbewegung ausführt. Die Kippbewegung des Kipphebels versetzt die Tätowiernadel in die Stichbewegung.

Tätowiermaschinen werden dazu verwendet, Motive (Tätowierungen) in Haut, insbesondere in menschliche Haut, zu stechen. Hierzu wird Tätowierfarbe mithilfe von Tätowiernadeln in die mittlere Hautschicht (Dermis) eingebracht. Dabei führt die Tätowiernadel eine Stichbewegung mit einer bestimmten Frequenz aus. Die Stichbewegung bezeichnet eine Aufwärts- und Abwärts-Bewegung der Tätowiernadel, die auch Schlag genannt wird.

Während des Tätowierens kann es sinnvoll sein, Parameter der Tätowiermaschine wie beispielsweise die Härte des Schlags, dass heißt die Härte, mit der die Tätowiernadel auf die Haut auftrifft bzw. in diese einsticht, zu variieren. Beispielsweise werden Linien eines Motivs in der Regel mit einem harten Schlag in die Haut gestochen. Farbige Flächen und Schattierungen werden in der Regel mit einem sehr weichen Schlag erstellt.

Grundsätzlich unterscheidet man drei Typen von Tätowiermaschinen.

Bei der Spulenmaschine wird durch die elektronmagnetische Kraft zweier Spulen ein Schlagarm in Bewegung versetzt, an dem eine Tätowiernadel befestigt ist. Der Schlagarm kann mit Federblechen unterschiedlicher Steifigkeit verbunden werden, über die die Härte des Schlags einstellbar ist. Die Spulenmaschine ist reparaturanfällig und wartungsintensiv. Zur Durchführung einer Reparatur oder Wartung ist technisches Fachwissen zwingend notwendig.

Die Rotarymaschine, als zweiter Typ von Tätowiermaschinen, hat einen Elektromotor, dessen rotatorische Bewegung über eine Exzenterscheibe und ein Pleuel auf einen Schlagarm übertragen wird. Der Schlagarm ist starr mit einer Tätowiernadel verbunden, die aufgrund der Antriebsbewegung des Elektromotors eine translatorische Bewegung ausführt. Die Härte des Schlags ist bei der Rotarymaschine nicht einstellbar, wodurch der Einsatzbereich dieser Tätowiermaschine begrenzt ist.

Ein dritter Typ von Tätowiermaschinen ist die sogenannte Hybridmaschine. Auch hier wird die Rotationsbewegung eines Antriebsmotors über Kurbelwelle und Pleuel in eine translatorische Bewegung der Tätowiernadel umgewandelt. Die Hybridmaschine weist einen Mechanismus auf, der auf eine Nadelhalterung, in der die Tätowiernadel gehalten wird, wirkt. Er verfügt über ein elastisches Bauteil wie beispielsweise einen Elastomer oder eine Druckfeder. Eine auf den Elastomer oder die Druckfeder wirkende Vorspannung ist einstellbar. Die Kraft, mit der der Elastomer oder die Druckfeder auf die Nadelhalterung wirkt, lässt sich justieren. Damit kann die Härte des Schlags variiert werden.

Allerdings ist es durch Variation der Vorspannung lediglich möglich, die Kraft einzustellen, ab der der Elastomer oder die Feder eine Stauchung erfährt. Die Kennlinie des Elastomers oder die Federkennlinie hingegen bleiben unverändert. Die Schlageigenschaften der Hybridmaschine können deshalb nur begrenzt eingestellt werden.

Siehe zum Beispiel EP 1958659 A1 oder FR 2972933 A1.

Aus oben genannten Gründen ergibt sich die Aufgabe, eine wartungsarme Tätowiermaschine mit einer optimierten Stichbewegung vorzuschlagen.

Bei der Lösung dieser Aufgabe wird von einer Tätowiermaschine gemäß dem Oberbegriff des Anspruchs 1 ausgegangen. Gelöst wird die Aufgabe durch die im Kennzeichen des Anspruchs 1 angegebenen konstruktiven Merkmale.

Der Kipphebel ist über eine Biegefeder mit der Nadelaufnahme verbunden. Die Antriebsbewegung des Antriebsmotors wirkt starr auf den Kipphebel. Von dort wird sie über die elastische Biegefeder auf die Nadelaufnahme und damit auf die Tätowiernadel übertragen. Bevorzugt ist die Tätowiernadel in Biegerichtung der Biegefeder, dass heißt quer zur Längsrichtung der Biegefeder angeordnet.

Die Steifigkeit der Biegefeder ist einstellbar, so dass die Härte der Stichbewegung variierbar ist. Wird die Steifigkeit der Biegefeder erhöht, sind Relativbewegungen zwischen Kipphebel und Nadelaufnahme bzw. Tätowiernadel nur eingeschränkt möglich. In diesem Fall arbeitet die Tätowiermaschine mit einem harten Schlag. Erfolgt eine Verringerung der Steifigkeit der Biegefeder, können große Relativbewegungen zwischen Kipphebel und Nadelaufnahme bzw. Tätowiernadel auftreten. In diesem Fall arbeitet die Tätowiermaschine mit einem weichen Schlag. Es ist denkbar, die Biegefeder zu blockieren, dass heißt die Steifigkeit der Biegefeder derart zu erhöhen, so dass die Antriebsbewegung des Antriebsmotors starr oder annähernd starr auf die Nadelaufnahme übertragen wird. Der Schlag ist dann im einstellbaren Bereich am härtesten.

Im Rahmen der Erfindung wurde erkannt, dass der Einsatzbereich einer Tätowiermaschine vergrößert wird, wenn die Härte des Schlags über einen möglichst großen Bereich verändert werden kann. Linien können dann besonders exakt gezeichnet und Flächen besonders gut ausgefüllt werden. Bevorzugt werden die Linien mit einer hohen Schlagfrequenz und Flächen mit einer geringen Schlagfrequenz gestochen.

Mit dem Begriff Härte wird im Rahmen der Erfindung die Nachgiebigkeit bezeichnet, mit der die Tätowiernadel auf die Haut auftrifft. Die Haut bietet beim Auftreffen der Tätowiernadel einen gewissen Widerstand, der in Abhängigkeit von der Hautpartie variieren kann. Wird die Stichbewegung der Tätowiernadel aufgrund dieses Widerstands behindert und dadurch die Kippbewegung des Kipphebels nicht vollständig in die Stichbewegung der Tätowiernadel umgesetzt, so wird im Rahmen der vorliegenden Erfindung von einer Nachgiebigkeit gesprochen. Es kommt dann zu Relativbewegungen zwischen dem starr angetrieben Kipphebel und der Tätowiernadel. Bei einer großen Härte, dass heißt bei einem harten Schlag, ist die Nachgiebigkeit der Tätowiernadel beim Auftreten auf die Haut sehr gering. Bei einer geringen Härte, das heißt bei einem weichen Schlag, ist die Nachgiebigkeit der Tätowiernadel beim Auftreffen auf die Haut sehr groß. Der Begriff Nachgiebigkeit ist im Rahmen der Erfindung gleichbedeutend mit einer Federsteifigkeit einer virtuellen Feder, die in Richtung der Stichbewegung der Tätowiernadel wirkt.

Es ist vorteilhaft, den Kipphebel als Lagerung für die Biegefeder auszubilden. Beispielsweise ist die Biegefeder einseitig durch den Kipphebel gelagert und an einem gegenüberliegenden Ende mit der Nadelaufnahme verbunden.

Bevorzugt ist der Kipphebel als Gehäuse ausgebildet, in dessen Innern die Biegefeder zumindest teilweise verläuft. Die Biegefeder wird somit vor äußeren Einflüssen geschützt. Beschädigungen werden vermieden. Beispielsweise ist die Biegefeder an einem Ende mit dem Gehäuse verbunden, dass heißt die Biegefeder ist bevorzugt einseitig eingespannt.

In einer weiteren bevorzugten Ausführungsform umfasst die Tätowiermaschine einen Hemmschlitten zum Hemmen von Federbewegungen der Biegefeder. Bevorzugt ist der Hemmschlitten in dem Gehäuse angeordnet und ermöglicht ein Einstellen der Steifigkeit der Biegefeder in Richtung der Stichbewegung. Die Steifigkeit einer Biegefeder ist abhängig von der Länge, über die sich die Biegefeder ungehindert verformen kann. Bei einer einseitig eingespannten Biegefeder ist dies der Abstand zwischen einem frei beweglichen Federende und der Einspannungsstelle der Biegefeder. Bevorzugt ist es mit dem Hemmschlitten möglich, die Länge, über die sich die Biegefeder ungehindert verformen kann, also die frei verformbare Federlänge, zu variieren. Beispielsweise dient der Hemmschlitten dazu, die Biegefeder an einer weiteren Position im Gehäuse zu lagern und so die frei verformbare Federlänge zu verändern. Indem die Steifigkeit der Biegefeder in Richtung der Stichbewegung einstellbar ist, kann mithilfe des Hemmschlittens die Härte des Schlags gezielt variiert werden. Hinsichtlich der obigen Definition des Begriffs der Härte, wirkt die Biegefeder also als virtuelle Feder in Richtung der Stichbewegung.

Es ist zweckmäßig, dass der Hemmschlitten in Längsrichtung der Biegefeder verschiebbar ist. Somit kann die Position des Hemmschlittens relativ zu der Biegefeder verändert werden. Es sind unterschiedlichste Positionen entlang der Längsrichtung der Biegefeder denkbar, an denen der Hemmschlitten als zusätzliche Lagerung bzw. Lagerstelle der Biegefeder dient. Die frei verformbare Federlänge der Biegefeder kann so mithilfe des Hemmschlittens verkürzt oder verlängert werden. Bevorzugt ist es möglich, den Hemmschlitten stufenlos in Längsrichtung der Biegefeder zu verschieben. Damit kann die Steifigkeit der Biegefeder und damit die Härte des Schlags stufenlos eingestellt werden.

Vorteilhafterweise weist der Hemmschlitten eine Aufnahme zur Lagerung der Biegefeder auf. Bevorzugt ist die Aufnahme derart ausgebildet, dass sie die Biegefeder an einer Position entlang deren Längsrichtung fixiert. Der Hemmschlitten wirkt somit zusammen mit der Aufnahme als weitere Einspannungsstelle der Biegefeder.

Weiter bevorzugt ist die Aufnahme als Durchgangsbohrung ausgebildet, durch die die Biegefeder hindurchragt. Ist die Biegefeder beispielsweise stabförmig oder zumindest teilweise stabförmig ausgebildet, umgreift die Aufnahme die Biegefeder entlang deren Umfangsrichtung zumindest in einem Teilabschnitt der Biegefeder. Bevorzugt sind die Abmessungen der Durchgangsbohrung derart gewählt, dass der Hemmschlitten mit der Durchgangsbohrung relativ zu der Biegefeder verschiebbar ist, die Durchgangsbohrung gleichzeitig aber eine Fixierung der Biegefeder ermöglicht.

Auch kann an dem Hemmschlitten ein Betätigungselement angeordnet sein, über das der Hemmschlitten in dem Gehäuse verschiebbar ist. Dadurch wird ein Verschieben des Hemmschlittens relativ zu der Biegefeder vereinfacht. Beispielsweise kann das Betätigungselement derart ausgebildet sein, dass es den Hemmschlitten in der jeweiligen Position entlang der Längsrichtung der Biegefeder arretiert. Der Hemmschlitten kann somit nicht selbständig in dem Gehäuse verfahren.

Bevorzugt weist das Gehäuse eine Öffnung auf, durch die das Betätigungselement aus dem Gehäuse herausragt. Für den Bediener ist es somit auf eine einfache Art und Weise möglich, den Hemmschlitten von der Außenseite des Gehäuses aus zu verschieben und damit die Steifigkeit der Biegefeder zu variieren. Dies erhöht den Bedienungskomfort der Tätowiermaschine.

Besonders bevorzugt ist die Biegefeder als Federstab ausgebildet. Beispielsweise besteht der Federstab aus Federstahl. Federstahl weist eine lineare Federcharakteristik auf, die ein besonders genaues Einstellen des Schlags bzw. der Schlageigenschaften über einen breiten Einstellbereich ermöglicht.

Die Erfindung wird nachfolgend anhand von einer in den Figuren dargestellten bevorzugten Ausführungsform erläutert. Die darin dargestellten Besonderheiten können einzeln oder in Kombination verwendet werden, um bevorzugte Ausgestaltungen der Erfindung zu schaffen. Die beschriebene Ausführungsform stellt keine Einschränkung der Allgemeinheit des in den Ansprüchen definierten Gegenstands dar. Es zeigen:
- Figur 1: eine erfindungsgemäße Tätowiermaschine mit einer Getriebeeinheit in einer perspektivischen Ansicht;
- Figur 2: einen Teil der Getriebeeinheit aus Fig. 1 in einer perspektivischen Ansicht.

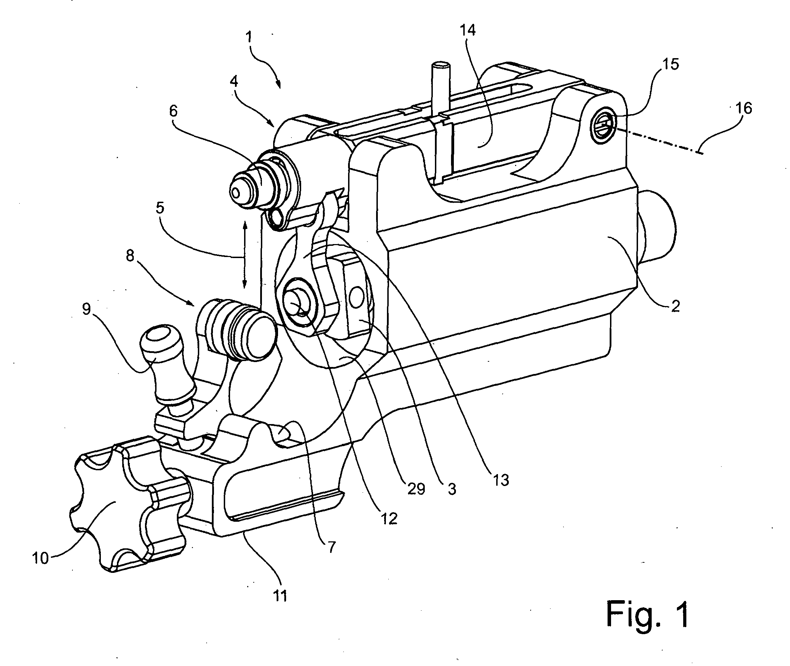

Figur 1 zeigt eine erfindungsgemäße Tätowiermaschine 1 zum Bewegen einer Nadel, insbesondere einer Tätowiernadel (nicht dargestellt). Die Tätowiermaschine 1 umfasst einem Rahmen 2 mit einer zylinderförmigen Motoraufnahme 29, in der ein Antriebsmotor 3 angeordnet und befestigt ist.

Eine rotatorische Antriebsbewegung des Antriebsmotors 3 wird mithilfe einer Getriebeeinheit 4 in eine Stichbewegung der Tätowiernadel entlang der Richtungspfeile 5 gewandelt. In der Stichbewegung führt die Tätowiernadel eine translatorische Aufwärts- und Abwärts-Bewegung aus und dringt dabei in die menschliche Haut ein.

In einem einsatzbereiten Zustand der Tätowiermaschine 1 ist die Tätowiernadel in einer Nadelaufnahme 6 befestigt und ragt durch ein Loch 7 durch den Rahmen 2 der Tätowiermaschine 1 hindurch. Mithilfe einer Nadelführung 8 wird die Tätowiernadel in ihrer Stichbewegung entlang der Richtungspfeile 5 geführt. Die Kraft, mit der die Nadelführung 8 auf die Tätowiernadel einwirkt kann über eine Einstellschraube 9 angepasst werden.

In den Rahmen 2 ist weiter eine Fixierschraube 10 einschraubbar, mit der ein Griff (nicht dargestellt) an einer Unterseite 11 des Rahmens 2 befestigt werden kann. Der Griff ermöglicht ein Halten der Tätowiermaschine 1 während des Tätowierens.

Die Getriebeeinheit 4, mit der die Antriebsbewegung des Antriebsmotors 3 in eine translatorische Stichbewegung der Tätowiernadel entlang der Richtungspfeile 5 gewandelt wird, umfasst eine Exzenterscheibe 12 und ein Pleuel 13. Das Pleuel 13 ist mit einem als Gehäuse 14 ausgebildeten Kipphebel, der ebenfalls Teil der Getriebeeinheit 4 ist, verbunden. Das Gehäuse 14 ist an dem Rahmen 2 an einer Lagerung 15 drehbar befestigt. Angetrieben durch die Antriebsbewegung des Antriebsmotors 3 führt das Gehäuse 14 eine Kippbewegung um die Achse 16 relativ zu dem Rahmen 2 aus. Die Kippbewegung des Gehäuses 14 versetzt die Tätowiernadel über die Nadelaufnahme 6 in die Stichbewegung entlang der Richtungspfeile 5.

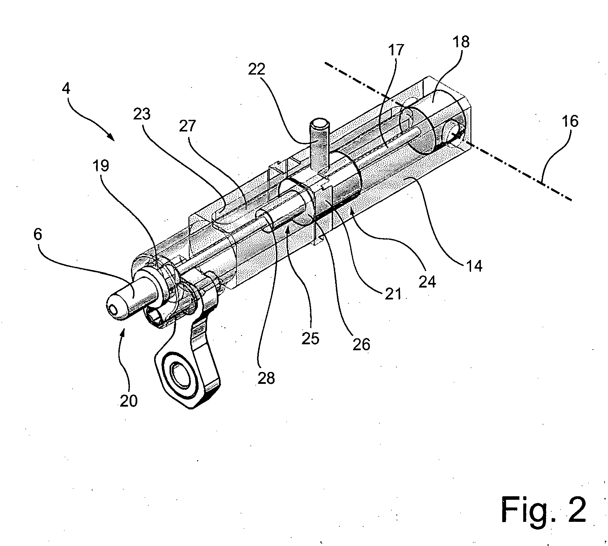

Gemäß Figur 2 ist das Gehäuse 14 hierzu über eine als Federstab 17 ausgebildete Biegefeder mit der Nadelaufnahme 6 verbunden. Der Federstab 17 ist Teil der Getriebeeinheit 4. Er erstreckt sich zumindest teilweise im Innern des Gehäuses 14. Im Ausführungsbeispiel gemäß Figur 2 sind mehr als dreiviertel der Länge des Federstabs 17 im Innern des Gehäuses 14 angeordnet.

An einem der Nadelaufnahme 6 gegenüberliegenden Ende des Federstabs 17 ist dieser mithilfe eines Einspannelements 18 einseitig im Gehäuse 14 eingespannt. Das Gehäuse 14 weist ein Langloch 19 auf, durch das ein frei schwingendes Ende 20 des Federstabs 17 aus dem Gehäuse 14 herausragt. Auf das frei schwingende Ende 20 des Federstabs 17 ist die Nadelaufnahme 6 aufgesteckt und fixiert. Die Nadelaufnahme 6 ragt teilweise in das Langloch 19 und damit in das Gehäuse 14 hinein. Die Nadelaufnahme 6 schlägt dadurch bei einer maximalen Auslenkung an dem Gehäuse 14 an. Somit ist die maximale Auslenkung der Nadelaufnahme 6 relativ zu dem Gehäuse 14 begrenzt.

Im Innern des Gehäuses 14 ist ein Hemmschlitten 21 zum Hemmen von Federbewegungen des Federstabs 17 angeordnet. Der Hemmschlitten 21 umfasst ein als Stift 22 ausgebildetes Betätigungselement, das quer zur Längsrichtung des Federstabs 17 angeordnet ist. Der Stift 22 ragt durch eine ovale Öffnung 23 im Gehäuse 14 aus diesem heraus.

Der Hemmschlitten 21 ist im Gehäuse 14 in Längsrichtung des Federstabs 17 verschiebbar angeordnet. Ein Verschieben des Hemmschlittens 21 kann durch Betätigung des Stifts 22 erfolgen. Um ein Verfahren des Hemmschlittens 21 im Innern des Gehäuses 14 zu ermöglichen, verfügt der Hemmschlitten 21 über einen ersten zylinderförmigen Abschnitt 24 mit einem ersten Durchmesser. Ein zweiter Abschnitt 25 des Hemmschlittens 21 schließt sich an den ersten Abschnitt 24 nahtlos an. Der zweite Abschnitt 25 ist ebenfalls zylinderförmig ausgebildet und weist einen zweiten Durchmesser auf. Der zweite Durchmesser des zweiten Abschnitts 25 ist kleiner als der erste Durchmesser des ersten Abschnitts 24 gewählt, so dass der Hemmschlitten 21 einen Absatz 26 zwischen dem ersten Abschnitt 24 und dem zweiten Abschnitt 25 aufweist.

Eine Innenwand 27 des Gehäuses 14 ist entsprechend der zylindrischen Außenkontur des ersten Abschnitts 24 des Hemmschlittens 21 geformt. Durch die aneinander angepassten Geometrien des Hemmschlittens 21 und der Innenwand 27, wird der Hemmschlitten 21 mittels der Innenwand 27 geführt. Durch die zylindrische Außenform des Hemmschlittens 21 wird ein Verkannten des Hemmschlittens 21 im Gehäuse 14 vermieden. Es versteht sich, dass zwischen der Außenkontur des ersten Abschnitts 24 und der Innenwand 27 minimales Spiel vorhanden ist, um ein Verschieben des Hemmschlittens 21 zu ermöglichen.

Der Hemmschlitten 21 weist eine Aufnahme zur Lagerung des Federstabs 17 auf. Gemäß Figur 2 ist diese Aufnahme als Durchgangsbohrung 28 ausgebildet. Die Durchgangsbohrung 28 erstreckt sich über die gesamte Längsrichtung des Hemmschlittens 21, dass heißt sowohl durch den ersten Abschnitt 24 als auch durch den zweiten Abschnitt 25. Der Federstab 17 ragt durch die Durchgangsbohrung 28 hindurch. Die Abmessungen der Durchgangsbohrung 28 sind derart gewählt, dass sie den Federstab 17 fixiert, gleichzeitig aber ausreichend Spiel vorhanden ist, um den Hemmschlitten 21 auf dem Federstab 17 zu verschieben.

Durch Verschieben des Hemmschlittens 21 in Längsrichtung des Federstabs 17 ist die Steifigkeit des Federstabs 17 einstellbar, so dass die Härte des Schlags, mit der die Tätowiernadel (nicht dargestellt) auf die menschliche Haut auftrifft bzw. in diese eindringt, variierbar ist. Ein Verschieben des Hemmschlittens 21 und damit ein Verstellen der Steifigkeit erfolgt bevorzugt durch Betätigen des Stifts 22 außerhalb des Gehäuses 14. Ein Verstellen der Steifigkeit des Federstabs 17 soll im Folgenden erläutert werden.

Die Steifigkeit des Federstabs 17 ist abhängig von der Federlänge, über die sich der Federstab 17 ungehindert verformen kann. Im Rahmen der Erfindung wird sie auch als die frei verformbare Federlänge bezeichnet. Im Falle des Federstabs 17 erstreckt sich die frei verformbare Federlänge ausgehend von dem frei schwingenden Ende 20 des Federstabs 17 bis zum Beginn der Durchgangsbohrung 28, dass heißt bis zum Beginn des zweiten Abschnitts 25 des Hemmschlitten 21.

Da eine Änderung der Steifigkeit des Federstabs 17 sämtliche Biegerichtungen des Federstabs betrifft, führt eine Veränderung der frei verformbaren Federlänge auch zu einer Veränderung der Steifigkeit des Federstabs 17 in Richtung der Stichbewegung der Tätowiernadel entlang der Richtungspfeile 5 (Figur 1).

Die frei verformbare Federlänge des Federstabs 17 ist durch Verschieben des Hemmschlittens 21 veränderbar. Der Hemmschlitten 21 wirkt dabei mittels der Durchgangsbohrung 28, durch die der Federstab 17 hindurchragt, als zusätzliche Einspannungsstelle des Federstabs 17. Er umgreift den sich durch die Durchgangsbohrung 28 erstreckenden Federstab 17 und hemmt so Federbewegungen des Federstabs 17. Gleichzeitig stützt sich der Hemmschlitten 21 über die Außenkontur des ersten Abschnitts 24 an der Innenwand 27 und damit am Gehäuse 14 ab. Der Federstab 17 ist also über den Hemmschlitten 21 mit dem Gehäuse 14 verbunden. Er greift folglich am Gehäuse 14 an. Befindet sich der Hemmschlitten 21 nahe der Achse 16 und stößt dort an das Einspannelement 18 an, ist die frei verformbare Federlänge des Federstabs 17 maximal. In dieser Position des Hemmschlittens 21 ist die Steifigkeit des Federstabs 17 minimal. Der Schlag mit dem die Tätowiermaschine 1 (Figur 1) arbeitet ist weich. Wird der Hemmschlitten 21 durch den Stift 22 in Richtung der Nadelaufnahme 6 verschoben, verkürzt sich die frei verformbare Federlänge des Federstabs 17 und die Steifigkeit des Federstabs 17 steigt. Der Schlag, mit dem die Tätowiermaschine 1 arbeitet wird härter.

Stößt der Hemmschlitten 21 mit seinem zweiten Abschnitt 25 an die Nadelaufnahme 6 an, ist die frei verformbare Federlänge des Federstabs 17 minimal und die Steifigkeit des Federstabs maximal. Der Schlag, mit dem die Tätowiermaschine 1 arbeitet ist hart.

Analog hierzu erfolgt das Verschieben des Hemmschlittens 21 zurück in Richtung des Einspannelements 18. Hierdurch wird die frei verformbare Federlänge des Federstabs 17 vergrößert. Die Steifigkeit des Federstabs 17 nimmt ab. Der Schlag der Tätowiermaschine 1 wird weicher.

Es versteht sich, dass der Hemmschlitten 21 im Innern des Gehäuses 14 arretiert werden kann. Somit wird vermieden, dass der Hemmschlitten 21 während des Gebrauchs der Tätowiermaschine 1 verschoben wird und sich die Steifigkeit des Federstabs 17 ungewollt ändert. Es ist denkbar in der Öffnung 23 Kerben oder Ausbuchtungen vorzusehen, in die der Stift 22 einrastet, um den Hemmschlitten 21 zu arretieren.

Eine Arretierung des Hemmschlittens 21 kann optional auch dadurch realisiert werden, dass der Hemmschlitten 21 in seiner Position relativ zu dem Federstab 17 mittels des Stifts 22 an dem Federstab 17 befestigt wird. Hierzu kann der Hemmschlitten 21 ein Innengewinde aufweisen, in das der Stift 22 eingeschraubt wird und das bis zum Federstab 17 reicht. Der Stift 22 kann soweit in das Innengewinde eingeschraubt werden, dass er auf den Federstab 17 drückt und den Hemmschlitten 21 am Federstab 17 fixiert.

Ebenfalls ist es denkbar, auf der Außenseite des Gehäuses 14 eine Skala aufzubringen, um einzelnen Positionen des Hemmschlittens 21 definierte Federsteifigkeiten des Federstabs 17 zuordnen zu können. Bevorzugt ist der Hemmschlitten 21 an Positionen arretierbar, die durch die Skala vorgegebenen sind.

## Patentansprüche

1. Tätowiermaschine zum Einbringen von Tätowierfarbe in menschliche Haut mithilfe einer Tätowiernadel, umfassend:
- einen Antriebsmotor (3) zum Antreiben der Tätowiernadel,
- eine Getriebeeinheit (4), die eine Antriebsbewegung des Antriebsmotors (3) in eine Stichbewegung der Tätowiernadel wandelt, mit der diese in die menschliche Haut eindringt, und
- eine Nadelaufnahme (6) zur Aufnahme der Tätowiernadel,
wobei die Getriebeeinheit (4) einen Kipphebel (14) aufweist, der durch die Antriebsbewegung des Antriebsmotors (3) eine Kippbewegung ausführt und,
wobei die Kippbewegung des Kipphebels (14) die Tätowiernadel in die Stichbewegung versetzt und,
wobei der Kipphebel (14) über eine Biegefeder (17) mit der Nadelaufnahme (6) verbunden ist,
**dadurch gekennzeichnet,**
**dass** die Steifigkeit der Biegefeder (17) einstellbar ist, sodass die Härte der Stichbewegung variierbar ist.

2. Tätowiermaschine nach Anspruch 1, **dadurch gekennzeichnet, dass** der Kipphebel als Gehäuse (14) ausgebildet ist, in dessen Inneren die Biegefeder (17) zumindest teilweise verläuft.

3. Tätowiermaschine nach Anspruch 2, **dadurch gekennzeichnet, dass** in dem Gehäuse (14) ein Hemmschlitten (21) zum Hemmen von Federbewegungen der Biegefeder (17) angeordnet ist, um die Steifigkeit der Biegefeder (17) in Richtung der Stichbewegung einzustellen.

4. Tätowiermaschine nach Anspruch 3, **dadurch gekennzeichnet, dass** der Hemmschlitten (21) in Längsrichtung der Biegefeder (17) verschiebbar ist.

5. Tätowiermaschine nach Anspruch 3, **dadurch gekennzeichnet, dass** der Hemmschlitten (21) eine Aufnahme (28) zur Lagerung der Biegefeder (17) aufweist.

6. Tätowiermaschine nach Anspruch 5, **dadurch gekennzeichnet, dass** die Aufnahme als Durchgangsbohrung (28) ausgebildet ist, durch die die Biegefeder (17) hindurchragt.

7. Tätowiermaschine nach Anspruch 3, **dadurch gekennzeichnet, dass** an dem Hemmschlitten (21) ein Betätigungselement (22) angeordnet ist, über das der Hemmschlitten (21) in dem Gehäuse (14) verschiebbar ist.

8. Tätowiermaschine nach Anspruch 7, **dadurch gekennzeichnet, dass** das Gehäuse (14) eine Öffnung (23) aufweist, durch die das Betätigungselement (22) aus dem Gehäuse (14) herausragt.

9. Tätowiermaschine nach einem der vorhergehenden Ansprüche, **dadurch gekennzeichnet, dass** die Biegefeder als Federstab (17) ausgebildet ist.

## Claims

1. Tattooing machine for introducing tattooing ink into human skin by means of a tattooing needle, comprising:
- a drive motor (3) for driving the tattooing needle,
- a transmission unit (4), which converts a driving movement of the drive motor (3) into a puncturing movement of the tattooing needle, causing said needle to penetrate the human skin, and
- a needle holder (6) to accommodate the tattooing needle,
wherein the transmission unit (4) has a toggle lever (14), which performs a tilting movement in response to the driving movement of the drive motor (3) and,
wherein the tilting movement of the toggle lever (14) actuates the tattooing needle into the puncturing movement and,
wherein the toggle lever (14) is connected via a bending spring (17) to the needle holder (6),
**characterised in that**
the stiffness of the bending spring (17) is adjustable so that the hardness of the puncturing movement is variable.

2. Tattooing machine according to claim 1, **characterised in that** the toggle lever is designed as a housing (14), through the inside of which the bending spring (17) passes at least partly.

3. Tattooing machine according to claim 2, **characterised in that** a blocking slide (21) for blocking spring movements of the bending spring (17) is arranged inside the housing (14) in order to adjust the stiffness of the bending spring (17) in puncturing movement direction.

4. Tattooing machine according to claim 3, **characterised in that** the blocking slide (21) is movable in the longitudinal direction of the bending spring (17).

5. Tattooing machine according to claim 3, **characterised in that** the blocking slide (21) has a retainer (28) for holding the bending spring (17).

6. Tattooing machine according to claim 5, **characterised in that** the retainer is designed as a through hole (28), through which the bending spring (17) protrudes.

7. Tattooing machine according to claim 3, **characterised in that** the blocking slide (21) comprises an actuating element (22), by means of which the blocking slide (21) is slidable inside the housing (14).

8. Tattooing machine according to claim 7, **characterised in that** the housing (14) has an opening (23), through which the actuating element (22) protrudes from the housing (14).

9. Tattooing machine according to any one of the preceding claims, **characterised in that** the bending spring is designed as a spring rod (17).

## Revendications

1. Machine à tatouer destinée à introduire de l'encre de tatouage dans la peau humaine à l'aide d'une aiguille de tatouage, comprenant :
- un moteur d'entraînement (3) servant à entraîner l'aiguille de tatouage,
- une unité de transmission (4) qui transforme un mouvement d'entraînement du moteur d'entraînement (3) en un mouvement de piqûre de l'aiguille de tatouage, permettant ainsi à cette dernière de pénétrer dans la peau humaine, et
- un logement d'aiguille (6) servant à recevoir l'aiguille de tatouage, l'unité de transmission (4) comportant un levier à bascule (14) qui exécute un mouvement de basculement grâce au mouvement d'entraînement du moteur (3), et
le mouvement de basculement du levier à bascule (14) mettant l'aiguille de tatouage en mouvement de piqûre, et
le levier à bascule (14) étant relié au logement d'aiguille (6) par l'intermédiaire d'un ressort de flexion (17),
**caractérisée en ce que**
la rigidité du ressort de flexion (17) est réglable de manière à faire varier la dureté du mouvement de piqûre.

2. Machine à tatouer selon la revendication 1, **caractérisée en ce que** le levier à bascule est réalisé sous forme de boîtier (14) à l'intérieur duquel le ressort de flexion (17) s'étend au moins partiellement.

3. Machine à tatouer selon la revendication 2, **caractérisée en ce que** dans le boîtier (14) est disposé un chariot de blocage (21) destiné à bloquer les mouvements du ressort de flexion (17) afin de régler la rigidité du ressort de flexion (17) dans le sens du mouvement de piqûre.

4. Machine à tatouer selon la revendication 3, **caractérisée en ce que** le chariot de blocage (21) peut être déplacé dans le sens longitudinal du ressort de flexion (17).

5. Machine à tatouer selon la revendication 3, **caractérisée en ce que** le chariot de blocage (21) comporte un logement (28) pour le montage du ressort de flexion (17).

6. Machine à tatouer selon la revendication 5, **caractérisée en ce que** le logement est réalisé sous forme de trou traversant (28) à travers lequel passe le ressort de flexion (17).

7. Machine à tatouer selon la revendication 3, **caractérisée en ce que** sur le chariot de blocage (21) est disposé un élément d'actionnement (22) au moyen duquel le chariot de blocage (21) peut être déplacé dans le boîtier (14).

8. Machine à tatouer selon la revendication 7, **caractérisée en ce que** le boîtier (14) comporte une ouverture (23) à travers laquelle l'élément d'actionnement (22) fait saillie du boîtier (14).

9. Machine à tatouer selon l'une des revendications précédentes, **caractérisée en ce que** le ressort de flexion est réalisé sous forme de tige à ressort (17).
